# EUROPEAN PATENT APPLICATION

(11) **EP 4 374 774 A1**
(43) Date of publication of application: **29.05.2024**
(21) Application number: 22209790.9
(22) Date of filing: 28.11.2022
(51) Int. Cl.: A61B 5/00

(54) **A SYSTEM AND METHOD FOR DETECTING MILK DUCT BLOCKAGE**

(71) Applicant: Koninklijke Philips N.V., 5656 AG Eindhoven (NL)
(72) Inventor: GOTTENBOS, Bart, Eindhoven (NL); MENA BENITO, Maria Estrella, Eindhoven (NL); JOHNSON, Mark Thomas, Eindhoven (NL); GERHARDT, Lutz Christian, Eindhoven (NL); DAMODARAN, Mathivanan, Eindhoven (NL); SEGAAR, Lucja Elzbieta, Eindhoven (NL); PAULUSSEN, Elvira Johanna Maria, Eindhoven (NL)
(74) Representative: Philips Intellectual Property & Standards

(57) **Abstract**

A system is provided for detecting milk duct blockage or partial blockage during application of a variable pressure to the breast. Captured optical images are processed to identify milk ducts and identify a geometry of the milk ducts over time. The milk duct geometry is compared between timings of first and second pressure levels of the variable pressure. Milk blockage can then be detected based on the comparison.

## Description

### FIELD OF THE INVENTION

This invention relates to the detection of milk duct blockage in a lactating breastfeeding mother.

### BACKGROUND OF THE INVENTION

Breastfeeding is crucial for a healthy development of a newborn baby. Indeed, the best source of nutrition for a baby is human milk given by a breastfeeding mother. The milk contains antibodies which help protect against many common childhood and future illnesses. The World Health Organization recommends to exclusively breastfeed for at least six months, meaning no other foods or liquids are provided.

During breastfeeding, the infant interacts with the nipple applying both vacuum cycles and tongue pressures to extract milk. Moreover, the infant stimulates the nipple and areola complex that leads to release of oxytocin and ejection of milk. The combination of the applied vacuum, the contracting response of the cells around the ducts (myoepithelial cells) to oxytocin release and the pressure of milk flowing through the ducts leads to opening of the milk ducts and release of milk.

In this process, the milk ducts show a highly dynamic behavior, going from a collapsed to a swollen milk filled state. Ultrasound imaging studies have shown that the milk duct size is highly responsive to pressure fluctuations (which also happens during the use of breast pumps).

However, breastfeeding is often halted due to breastfeeding related breast pain and breast traumas caused by the act of breastfeeding and/or milk expression itself. Pain and trauma are among the main causes of weaning from breastfeeding.

For example, mastitis is a common condition occurring during breastfeeding, being a painful inflammation of the breast tissue. It is most often caused by clogged milk ducts that lead to milk stasis (remaining milk in the breast after a feed giving a restricted milk flow), sometimes in combination with infectious bacteria. Clogged milk ducts refer to milk ducts with some obstruction, e.g. caused by milk clutters. They can be painful, feel warm, cause discomfort and as stated above lead to the development of mastitis.

Clogged milk ducts can be released by applying massage, warmth or breastfeeding and/or pumping on a more frequent basis to open blocked ducts. Some signs can be predictive of clogged ducts, including localized redness of the skin, a hard or semi-hard lump on the breast, and a feeling of pain during milk ejection reflex.

However, due to the many various symptoms which can result from breastfeeding (like damaged nipple, blebs, abscesses and more) mothers find it difficult to recognize and identify the right condition and, as consequence, do not timely apply the most suitable treatment preventing the development of more serious conditions and eventually, weaning from breastfeeding.

In clinical breastfeeding research, ultrasound-based imaging technologies are used to image milk ducts. This imaging method would be difficult to translate to a home solution, since ultrasound requires a coupling between the ultrasound transducer and the tissue, e.g., using an ultrasound gel, which is inconvenient and also not desirable as it may end up in the milk. Also, ultrasound does not have a high enough resolution to see narrower sections of the ducts (duct endings are of the order of 0.07 mm in size).

Currently, there are no consumer-friendly solutions for early detection of clogged ducts. The only methods relate to visual or physical judgment of the mother herself or trained professional. Those methods, however, come often late or are very subjective.

A technology to detect clogged milk ducts early and which is suitable for consumer use would be of interest, so the mother can clear the clogged ducts on time to prevent pain, the development of mastitis and eventually, prevent weaning from breastfeeding.

### SUMMARY OF THE INVENTION

The invention is defined by the claims.

According to examples in accordance with an aspect of the invention, there is provided a system for detecting milk duct blockage during application of a variable pressure to the breast, comprising:
an optical imaging system comprising a light source and a light detector,
a processor for processing the captured images, wherein the processor is configured:
   to identify milk ducts in the images;
   to identify a geometry of the milk ducts over time;
   to receive timing information in respect of a variable pressure applied to the breast or identify timing information in respect of a variable pressure applied to the breast, the variable pressure having at least first and second pressure levels;
   to compare a milk duct geometry between timings of the first and second pressure levels; and
   determine milk blockage based on the comparison.

The milk duct blockage may be a partial or a full blockage.

The applied pressure may be positive (so that there is an atmospheric pressure and an increased pressure) or negative (so that there is an atmospheric pressure and a reduced pressure i.e. a partial vacuum), or both pressure levels may be different to atmospheric pressure (e.g. two increased pressures, or two reduced pressures, or one above atmospheric pressure and one below).

This system makes use of optical imaging to capture images of milk ducts. The milk ducts can be identified in the images and their geometry can thus be analyzed. The milk ducts change geometry in response to the application of pressure (positive or negative) to the breast. This variable pressure may be generated by a breast pump, or by natural breastfeeding, or it may be a pressure applied by a breast massage device or any other device.

The change in geometry (such as duct area or volume or duct diameter) in response to different pressures is indicative of different milk duct conditions, and in particular can be used to detect milk duct blockages or partial blockages.

The light source and light detector are for example for positioning on opposite sides of the nipple.

The variable pressure for example comprises a cyclic pressure. A cyclic pressure is for example generated by a breast pump, but even natural breastfeeding has a cyclic behavior.

The first and second pressure levels may comprise a pressure peak and a pressure trough. There may be a single peak and a single trough for the purposes of the imaging, or multiple peaks and troughs may already exist as part of a cyclic pressure waveform. It is instead possible to analyze the geometry at intermediate timings, so not necessarily at the exact peaks and troughs of a cyclic waveform.

The milk duct geometry may comprise a milk duct diameter. This may be measured as a simple linear dimension within a captured image.

The processor may be configured to compare the milk duct geometry by obtaining a milk duct diameter ratio. The ratio between the milk duct diameter for the peak and trough pressures provides a measure of the duct compliance, and this may be used to determine when there is a milk duct blockage.

The processor may be configured to determine a milk blockage by comparing the milk duct diameter ratio with a threshold or with previous milk duct diameter ratios for the user. Thus a predetermined default value of the ratio may apply, or else the system may look for changes over time in duct response for a particular user, thereby personalizing the detection to individual users.

The processor may be configured to identify individual ducts and store milk duct geometry information for identified the ducts. This may then be used to provide an analysis of the change in individual duct characteristics over time.

The optical imaging system may be for capturing images from two or more directions between opposite sides of the nipple of the user of the system. In this way, milk ducts may be viewed from different directions, e.g. orthogonal directions, to enable a more three dimensional assessment of the milk duct geometry, for example enabling a more accurate assessment of the milk duct diameter.

In one example, the light source comprises multiple light source elements and the light detector comprises multiple light detector elements, to provide the different imaging directions. This is a simple implementation but requires multiple components.

In another example, the light source and the light detector are moved between different orientations, to provide the different imaging directions. This reduces the required components but makes the operation of the system more complicated, requiring more user involvement (for manual rotation) or an additional actuation system (for automated rotation).

In another example, the optical imaging system is rotatable and is configured to perform a scan of different imaging directions. This may enable a more complete 3D image to be formed.

The processor may be configured to generate feedback indicating an area at which a blocked duct has been identified. This enables the user to take corrective curative action.

The light source for example comprises a near infra red light source. Infra red light may have sufficiently low scattering and absorption to penetrate the nipple fully. Based on scattering differences between milk and tissue a visual contrast between ducts and the surrounding tissue is obtained. Specific wavelengths may be chosen which have a difference in absorption between the milk and the breast/nipple tissue, for example using absorption of fatty acids in milk or other milk specific components.

In one set of examples, the system is for detecting milk duct blockage during breastfeeding, wherein the variable pressure is the pressure generated by the infant during breastfeeding.

The invention then also provides a nipple shield for use during breastfeeding, wherein the nipple shield comprises the system defined above for detecting milk duct blockage.

In another set of examples, the system is for detecting milk duct blockage during breast pumping, wherein the variable pressure is the pressure generated by the breast pump.

The invention then also provides a breast pump comprising:
a pressure source for generating a cyclic pressure; and
a breast shield for applying the cyclic pressure to the breast; and
the system for detecting milk duct blockage as defined above.

The invention also provides a computer-implemented method for detecting milk duct blockage during application of a cyclic pressure cycle to the breast, comprising:
receiving captured images of at least the nipple of a user;
receiving timing information for a variable pressure applied to the breast or identifying timing information for a variable pressure applied to the breast, the variable pressure having at least first and second pressure levels;
processing the images, thereby:
   to identify milk ducts in the images;
   to identify a geometry of the milk ducts over time;
   to compare a milk duct geometry between timings of the first and second pressure levels; and
   to determine milk blockage based on the comparison.

A computer program comprises computer program code which is adapted, when said program is run by the processor of the system defined above, to implement this method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 a pressure cycle generated by a breast pump as well as milk duct geometry at different times during the pressure cycle;
Fig. 2 shows an optical imaging system for imaging milk ducts;
Fig. 3 shows a plot of the ratio of absorption coefficient of a fatty substance to water (y-axis) versus wavelength;
Fig. 4 shows a processing method for detecting milk duct blockage during application of a cyclic pressure to the breast; and
Fig. 5 shows that the system may be integrated with a nipple shield or the breast shield of a breast pump.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for detecting milk duct blockage or partial blockage during application of a variable pressure to the breast. Captured optical images are processed to identify milk ducts and identify a geometry of the milk ducts over time. The milk duct geometry is compared between timings of first and second pressure levels of the variable pressure. Milk blockage can then be detected based on the comparison.

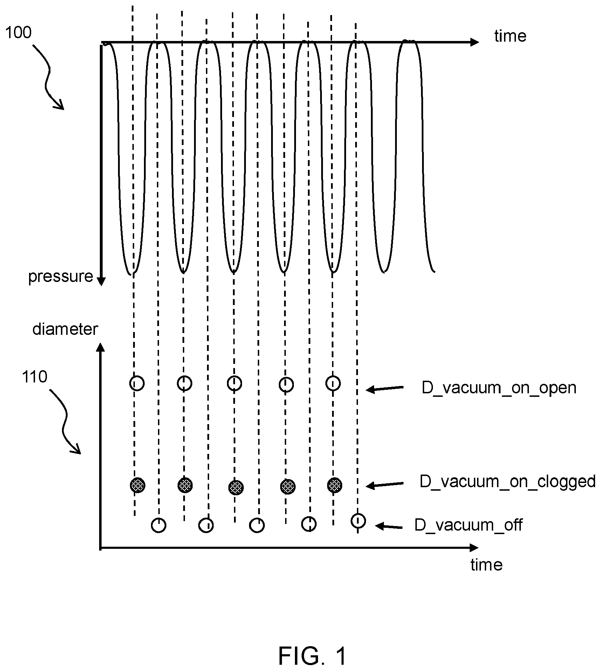

Fig. 1 shows as plot 100 a pressure cycle generated by a breast pump. The pressure cycle alternates between zero pressure (by which is meant atmospheric pressure) and a negative pressure (i.e. a vacuum pressure level). This cyclic pressure waveform is one example of a variable pressure. As will be clear from the discussion below, the invention only needs two different pressure levels at which geometry assessments are made. They may or may not be part of a cyclic pressure waveform.

The invention makes use of dynamic imaging of the milk ducts during the changes in pressure between the at least two pressure levels, such as the breast pump pressure fluctuations explained above, around the nipple-areola region.

Dynamic imaging of the milk duct sizes is able to detect clogged ducts, as such ducts downstream of a blockage will minimally respond to the pressure fluctuations as the milk ejected from the glands cannot enter these ducts to enlarge them during a vacuum cycle. Milk ducts upstream of a blockage will show a different swelling and collapsing behavior, where they still enlarge upon application of the vacuum pressure, but do not empty as quickly at elevated pressures. In this way, clogged ducts can be identified early, and lactating women can take the appropriate measures to clear the clogged ducts in order to prevent mastitis.

Fig. 1 also shows as plot 110 a set of duct diameter measurements at different times during the pressure cycle of a breast pump.

A first set of points shows the diameters when the vacuum is off, i.e. when atmospheric pressure is applied. The same duct diameter is observed for clogged ducts as for open ducts ("D_vacuum-off'). A second set of points shows the diameters when the vacuum is on, and for clogged milk ducts ("D_vacuum_on_clogged"). A third set of points shows the diameters when the vacuum is off, and for open milk ducts ("D_vacuum_on_open").

The duct diameter is relatively small at relatively high pressure (atmospheric pressure in this case) and relatively large at relatively low pressure (the vacuum pump pressure in this case).

The duct diameter thus increases when the vacuum is applied. The increase is however dramatically lower if the duct is clogged. When the vacuum is off the pressure is high and the duct diameter small in both cases.

The invention is based on detecting the milk duct size and structure using optical imaging, in particular using transillumination of the nipple-areola region (the teat) using light (e.g. near infrared (NIR) light) on one side and a camera in the form of an electromagnetic sensor array on the other side.

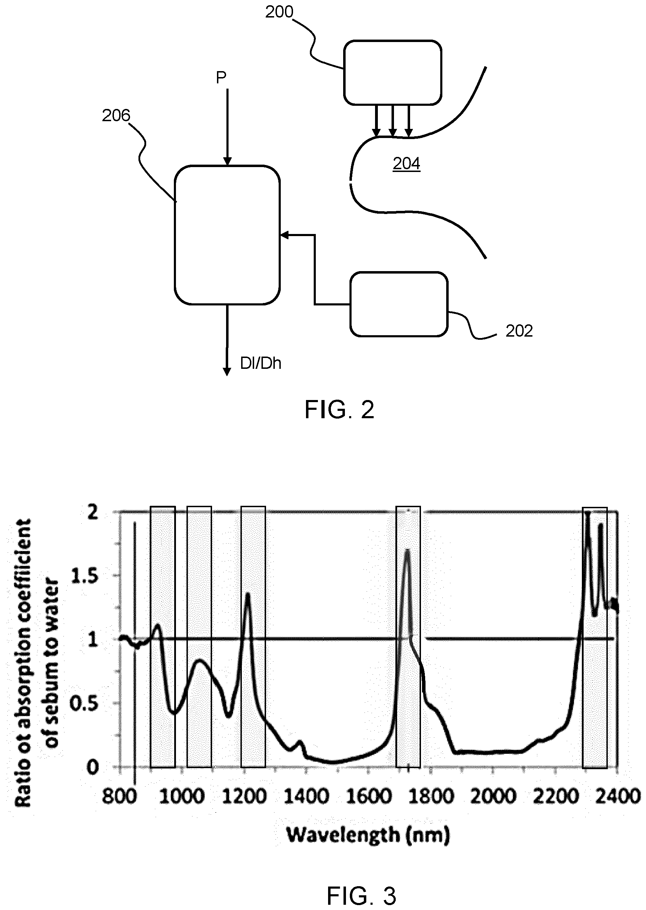

Fig. 2 shows the optical imaging system comprising a light source 200 and a light detector 202, for positioning on opposite sides of the nipple 204 of a user of the system. A processor 206 is provided for processing the captured images.

The optical imaging system may provide images of the nipple and optionally also a portion of the breast behind the nipple/areola.

Suitable wavelengths may be chosen which have sufficiently low scattering and absorption to penetrate the teat fully, such as (near) infrared wavelengths. Based on scattering differences between milk and tissue, a visual contrast between ducts and the surrounding tissue is obtained. To further optimize contrast between ducts and teat tissue specific wavelengths may be chosen which have a difference in absorption between the two, e.g. wavelengths absorbed by fatty acids in the milk.

IR is suitable for structural duct imaging as the wavelengths can penetrate deep into the tissue and differentiate milk-filled ducts from surrounding tissue. Visible light may not have sufficient penetration depth to obtain a sufficient transmitted signal through the nipple, but longer wavelengths, such as near infrared or infrared wavelengths would be able to penetrate and generate an image on the camera. Milk has very different scattering properties compared to the surrounding breast tissue, which can provide a good contrast to visualize the duct internal geometry (size and shape). A suitable solution could for example be a 750nm LED and a normal CMOS camera which is still sensitive in that wavelength area.

Due to the different absorption coefficients of milk (lipids and water) and water alone, simple contrast imaging and image processing can be used to enhance visualization of ducts. For example, fat content is relatively high in milk, which absorbs for example more compared to water at wavelengths around 1720nm and 2300nm.

Fig. 3 shows a plot of the ratio of absorption coefficient of a fatty substance to water (y-axis) versus wavelength (x-axis). Wavelengths at which there are peaks are shown in rectangles. To enhance contrast at lower wavelengths a possible strategy is to use two wavelengths e.g., 800nm and 1200nm, 1200nm and 1400nm or 1064nm and 1450nm, the latter being wavelengths with relatively cheap sources available. This enables relative contrast imaging. Three wavelengths may be used for contrast-calibrated imaging taking into account absorption of soft tissue e.g., 800nm, 1000nm and 1200nm.

The detection technology may be built into already existing devices or products used by breastfeeding mother, such as a standard breast pump, wearable breast pump or a nipple shield. This has the advantage that in all cases a fluctuating pressure cycle is already present (generated by the breast pump or by the baby sucking) and the mother does not need to spend any additional time performing the detection.

The imaging is synchronized with the changes in pressure level of the variable pressure, for example synchronized with the cyclic pressure cycle, so that the duct geometry (e.g. diameter) is determined from images during a first pressure level (e.g. the minimum, trough, pressure) and a second pressure level (e.g. the maximum, peak, pressure) in the cycle, so that the duct diameters (or other geometry) as shown in Fig. 1 may be derived. This provides an optimal differentiation between open and closed ducts.

The processor 206 then compares a milk duct geometry between timings of the pressure peak and the pressure trough, i.e. it compares the diameter measurements D_vacuum_on_open (i.e. the high diameter Dh) with D_vacuum_on_clogged (i.e. the low diameter Dl).

One comparison is to find the ratio between the two values Dl/Dh. However, other values may be derived which are indicative of the change in duct geometry resulting from clogging of the milk duct.

The system is intended to be used repeatedly over time, e.g., every day. In this way, a sudden behavior change of a duct is the most reliable signal that blockage is occurring. Thus, each duct will have its own geometry when open and when clogged, and it is the change from one state to the other that is to be detected. To follow individual ducts over time the algorithm may be self-learning to recognize the ducts from specific features such as shape or size features, or using neural network algorithms.

A full spatial image of the ducts may be provided if the imaging is conducted from multiple directions, e.g. from two lines of sights perpendicular to each other. This could be achieved by rotating a single light source and camera feature, or by using multiple radially arranged light sources and sensor arrays.

To reduce the required penetration depth, the teat may be flattened by the transillumination system or the breast pump it is built into. There are commercially available breast pumps which include have nipple stimulating features which touch the teat. These could be used to perform this flattening function.

An enhanced tomographic imaging system may be used for example integrated to a breast shield of a breast pump. The imaging system may then obtain images from different directions or sections around the nipple in order to generate a full (semi)-3D picture of the ducts in the teat or breast section.

In a simple embodiment the imaging system may be integrated in a device, e.g., a breast pump, in such a way that it can be rotated around the nipple, either freely or under defined angles, e.g. lines of sight perpendicular to each other. The mother may simply rotate the imaging system to achieve this functionality, or the device may rotate itself automatically, e.g. driven by the vacuum pulses. In a freely rotatable system it may measure the rotation, e.g. using a gravitation sensor, in this way creating a tomographic image. Alternatively, in more complex embodiments two or more image sensors could be combined to create such an image.

Tracking duct geometry over time, e.g. from consecutive pumping sessions over consecutive days, can additionally be a powerful way to decide whether a duct is blocked. Each duct will have its own diameter and diameter fluctuation pattern and also has different unique features such as location in the breast, widening and narrowing patterns, branching patterns etc. Therefore, individual ducts may be recognized and followed over time. If the dynamic movement of a particular duct significantly drops, this indicates (partial) blockage, and the mother may be alerted to treat it. The algorithm may include learning features, such as neural networks to improve recognition of individual ducts.

A feedback system may also be provided indicating the areas to which attention is needed, so the mother can for example focus on this area by changing position of breastfeeding, feeding more frequently etc. This may be implemented as a wireless communication to a user device such as a smart phone, to provide a report which includes a graphical representation of the breast so that the area of blocked milk duct can be identified. Additionally, there may be alarm features such that the device automatically sends messages, blinking lights or certain sounds etc., to alarm the breastfeeding mother if clogged ducts are detected.

The example above is based on situations where a reduction in pressure (vacuum) is the cause of pressure fluctuation such as in a breast pump. Any cause of pressure fluctuation can be advantageously used in order to better identify clogging of ducts. Specifically, an increase of pressure will also cause a pressure fluctuation which is also capable of inducing the differential swelling of the ducts as a result of a (partial) blockage. For example, milk ducts upstream of a blockage will show a different swelling and collapsing behavior, where - instead of the usual contraction on a pressure increase - they actually enlarge close to the blockage as milk is forced towards the blockage and cannot easily pass through.

A device which causes a pressure increase could be a massage type of device, for example as used to treat mastitis, whereby also the effectiveness of the massage treatment can be followed, or specific massage modes may be used to optimally treat clogged ducts when detected. In fact the baby also produces a form of pressure increase during feeding with the (rolling) tongue and the jaws, which may also be picked up in a breast shield implementation.

The invention may be applied to a breast pump but it may also be used by mothers who breastfeed. The system can then be integrated into a nipple shield, often used in lactation. A baby will induce pressure cycles, which could be both vacuum cycles and positive pressure cycles combined.

In the case of a breast pump, the breast pump controller can provide a signal indicating the timing of the pressure waveform. Synchronizing with the pressure cycles during breastfeeding may be possible by estimating the sucking period and adapting the imaging timing accordingly. An additional pressure sensor may however also be used to enable optimal timing of the imaging system.

In alternative embodiments the imaging detector can be a stand-alone device for assessment of possible clogged ducts, to be used in combination with a separate system to induce suitable pressure cycles.

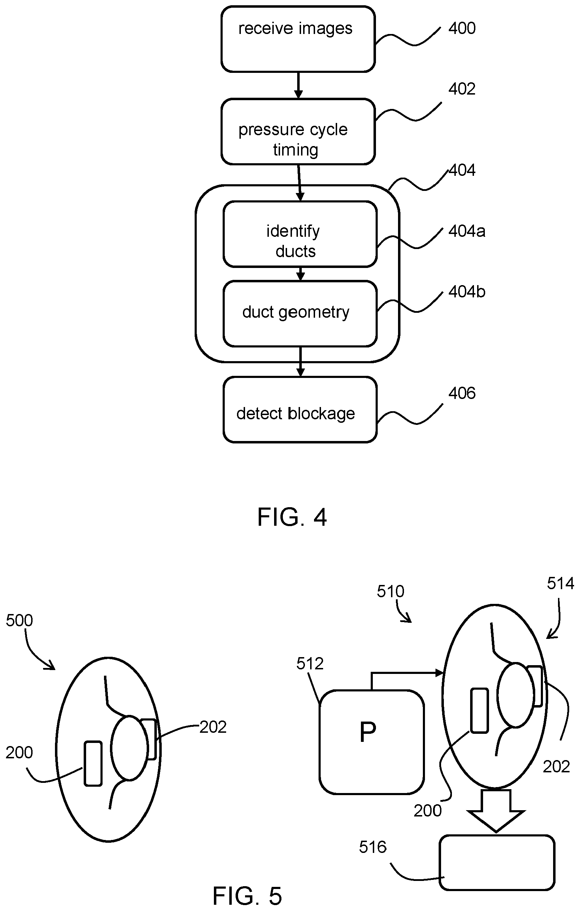

Fig. 4 shows the processing method for detecting milk duct blockage during application of a cyclic pressure to the breast.

In step 400, captured images of at least the nipple of a user are received. There may be a continuous stream of images, only some of which are selected for analysis, or images may only be captured at specific times, in particular the timing of a pressure peak and a pressure trough.

In step 402, applied pressure cycle timing information is received or else a pressure cycle applied to the breast is identified, the pressure cycle having the pressure peak and the pressure trough. The timing information may be received from the breast pump that generates the pressure cycle. Alternatively, it may be received from a pressure sensor. Alternatively, the image processing may extract the pressure cycle information based on the cyclic deformation of the milk ducts (the same cyclic deformation timing applies to clear and blocked milk ducts).

The typical sucking frequency is not very high, for example around 1 Hz, so a low video rate of for example 10 Hz would be sufficient to detect the low and high pressures.

In step 404 the images are processed. This involves identifying milk ducts in the images in step 402a, identifying a geometry of the milk ducts over time in step 402b. The processing does not need to be in real time. The images can for example be stored and analyzed after the breast pumping or breast feeding event, and the output may be given a short time afterwards. However, real time processing is also possible.

In step 404 a milk duct geometry is compared between timings of the pressure peak and the pressure trough. The peak may be a vacuum off time and the trough may be vacuum on time. Alternatively, they may be pressures generated by a baby during breastfeeding. The milk duct geometry may comprise a duct volume, area or diameter.

In step 406 milk blockage is detected based on the comparison. In a particular example, the comparison involves calculating a diameter ratio Dl/Dh as indicator for duct clogging.

Moreover, the algorithm can be expanded to calculate and represent/visualize the diameter ratio over time: Such tracking of diameter ratio over time may provide information on the viscoelastic duct properties and change in duct elasticity (fatigue).

Fig. 5 shows on the left shows a nipple shield 500 for use during breastfeeding, wherein the nipple shield comprises the system for detecting milk duct blockage. The processor is not shown. It is for example remote to the nipple shield, and the nipple shield relays the images to the remove processing device, e.g. smart phone.

Fig. 5 shows on the right schematically a breast pump 510 comprising a pressure source 512 for generating a cyclic pressure, a breast shield 514 for applying the cyclic pressure to the breast and a milk collection container 516.

Other parts of the breast pump are not shown, since no further modification is needed to a standard breast pump design. The breast pump may be wearable or it may be external.

The system for detecting milk duct blockage is integrated with the breast shield 514. The processor is again not shown. It is for example the main processor of the breast pump, or it may again be remote to the breast pump.

Another example is a stand alone device with no other function.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system for detecting milk duct blockage during application of a variable pressure to the breast, comprising:
an optical imaging system comprising a light source (200) and a light detector (204),
a processor (206) for processing the captured images, wherein the processor is configured:
to identify milk ducts in the images;
to identify a geometry of the milk ducts over time;
to receive timing information in respect of a variable pressure applied to the breast or identify timing information in respect of a variable pressure applied to the breast, the variable pressure having at least first and second pressure levels;
to compare a milk duct geometry between timings of the first and second pressure levels; and
determine milk blockage based on the comparison.

2. The system of claim 1, wherein the variable pressure comprises a cyclic pressure.

3. The system of claim 1 or 2, wherein the first and second pressure levels comprise a pressure peak and a pressure trough;

4. The system of any one of claims 1 to 3, wherein the milk duct geometry comprises a milk duct diameter.

5. The system of any one of claims 1 to 4, wherein the processor (206) is configured to compare the milk duct geometry by obtaining a milk duct diameter ratio.

6. The system of claim 5, wherein the processor (206) is configured to determine a milk blockage by comparing the milk duct diameter ratio with a threshold or with previous milk duct diameter ratios for the user.

7. The system of any one of claims 1 to 6, wherein the optical imaging system is for capturing images from two or more directions between opposite sides of the nipple of the user of the system.

8. The system of claim 7, wherein:
the light source comprises multiple light source elements and the light detector comprises multiple light detector elements, to provide the different imaging directions; or
the light source and the light detector are moved between different orientations, to provide the different imaging directions; or
the optical imaging system is rotatable and is configured to perform a scan of different imaging directions.

9. The system of any one of claims 1 to 8, wherein the light source (200) comprises a near infra red light source.

10. The system of any one of claims 1 to 9, for detecting milk duct blockage during breastfeeding, wherein the variable pressure is the pressure generated by the infant during breastfeeding.

11. A nipple shield for use during breastfeeding, wherein the nipple shield comprises the system for detecting milk duct blockage of claim 10.

12. The system of any one of claims 1 to 9, for detecting milk duct blockage during breast pumping, wherein the variable pressure is the pressure generated by the breast pump.

13. A breast pump comprising:
a pressure source for generating a cyclic pressure; and
a breast shield for applying the cyclic pressure to the breast; and
the system for detecting milk duct blockage of claim 12.

14. A computer-implemented method for detecting milk duct blockage during application of a cyclic pressure to the breast, comprising:
receiving captured images of at least the nipple of a user;
receiving timing information for a variable pressure applied to the breast or identifying timing information for a variable pressure applied to the breast, the variable pressure having at least first and second pressure levels;
processing the images, thereby:
to identify milk ducts in the images;
to identify a geometry of the milk ducts over time;
to compare a milk duct geometry between timings of the first and second pressure levels; and
to determine milk blockage based on the comparison.

15. A computer program comprising computer program code which is adapted, when said program is run by the processor of the system of claim 1, to implement the method of claim 14.
